# EUROPEAN PATENT APPLICATION

(11) **EP 4 491 780 A1**
(43) Date of publication of application: **15.01.2025**
(21) Application number: 23184712.0
(22) Date of filing: 11.07.2023
(51) Int. Cl.: D03D 1/00, D03D 13/00, D03D 15/533, A61B 5/00

(54) **A WOVEN FABRIC SENSOR FOR MULTI-DIMENSIONAL SENSING AND AN OPERATING METHOD FOR ANALYSING A SINGLE OUTPUT SIGNAL GENERATED BY A WOVEN FABRIC SENSOR**

(71) Applicant: Studio 1 Labs Danielewicz, Shim, Wegner Limited Joint-Stock Partnership, 02-962 Warsaw (PL)
(72) Inventor: Wegner, Karsten, 64395 Andreas-Bock-Str.10 (DE); Shim, Edward, Toronto, M2K 2Y6 (CA); PALACZ, Tomasz Andrzej, 81371 München (DE)
(74) Representative: Flügel Preissner Schober Seidel

(57) **Abstract**

The invention concerns a woven fabric sensor (100) for multi-dimensional sensing comprising: a plurality of electrically conductive warps (10a to 10f) arranged parallel to each other, and at least one electrically non-conductive weft (20a to 20i) floating over or under the warp (10), characterized in that the weft (20a to 20i) comprises a bridge section (22a to 22i) floating over or under at least two electrically conductive warps (10a to 10f) for generating an electrically connecting bridge path (30a to 30e). The invention concerns further an operating method for analysing a single output signal generated by a woven fabric sensor (100) carried out by a control unit (230), the method comprising the followings steps: a) receiving a single input sensor signal (Sᵢₙ1) having a signal value (SV1), b) analysing the signal value (SV1) for determining a magnitude of a sensing parameter, and c) generating a output signal (Sₒᵤₜ) indicating the magnitude of the sensing parameter.

## Description

The invention relates to a woven fabric sensor for multi-dimensional sensing, a multi-dimensional sensing system, and a seat or a bed comprising the woven fabric sensor for multi-dimensional sensing. The invention further relates to an operating method for analysing a single output signal generated by a woven fabric sensor.

The development of smart materials and smart sensors using electronic textiles with woven fabric sensors is emerging worldwide. The sensors commonly used use either capacitive or resistive sensing methods and are applied to a variety of cases such as seats in the automotive industry or beds in the healthcare industry.

Capacitive sensing generally uses two layers of conductive materials separated by a non-conductive layer, to transfer charge that bounces from one conductive layer to the other conductive layer to create a sensor signal change corresponding to electrical resistance. An equivalent exemplary fabric sensor hereto is disclosed in EP 3374551 A1 or WO 2018 076 106 A1.

However, this structure does not allow capacitive sensing to be used for voltage differential sensing, whereas voltage differential requires two conductive layers to come into contact for a transfer of charge, resulting in a sensor signal change corresponding to electrical resistance.

Conventional electronics and sensors, such as exemplarily disclosed in DE 11 2017 000 505 T5 or US 2005 072 250 A1, operate in a closed loop that includes power and ground. Through capacitive or voltage differential sensing methods, the resistance provides a changing signal which is then translated into a meaningful output information. To derive more meaningful information from an electrical signal from a single electrical source, a load is applied which requires additional parts and components. An example of an added load is a variable resistor to create a dynamic electrical signal to increase or decrease the energy flow in a closed loop, such as a dimmer switch that allows a light to be brightened or dimmed, by the use of added resistors. Electronic systems have a limited power supply. Adding extra parts and sensors to derive more information results in increased power consumption, with the amount of power required based on each load to function. The addition of extra parts and sensors increase the costs, complexity and functionality of electronic systems, the development process, and the dependence on diverse supply chains.

Another fabric sensor is known from WO 2009/093040 A1, but this fabric is knitted rather than woven. A knitted fabric generally has more stretch than a woven fabric. However, the excessive movement of most electrically conductive materials reduce their lifetime, as the conductive material tends to break when it is exposed to excessive movement.

Woven fabrics comprising an electrically conductive material are also known from EP 2 524 983 A2. However, this fabric consists of non-conductive warp and non-conductive weft yarns. The electrically conductive wire is an additional wire woven into the fabric additionally to the electrically non-conductive warp yarns and electrically non-conductive weft yarns.

A common drawback of fabric sensors is a high level of signal noise, which leads to higher power consumption and inaccuracy in signal analysis.

The object of the invention is to provide a woven fabric sensor, a multi-dimensional sensing system, a seat or a bed as well as an operating method with an improved energy efficiency and a reduced signal noise.

The object of the invention is solved by a woven fabric sensor according to independent claim 1, a multi-dimensional sensing system according to independent claim 10, a seat or a bed according to independent claim 11, an operating method according to claim 12 and a computer program product according to claim 15.

A first aspect of the invention is directed at the woven fabric sensor for multi-dimensional sensing, which comprises a plurality of electrically conductive warps and at least on electrically non-conductive weft. The warps are arranged parallel to each other. The at least one weft is floating over or under the warp. The weft comprises a bridge section floating over or under at least two adjacent electrically conductive warps for generating an electrically connected bridge path.

The bridge path presses at least two parallel warps together so that the warps are electrically connected. This results in an improved sensitivity of the sensor as the sensing area increases. As a result, the signal detected by the woven fabric sensor according to the invention is of greater magnitude and, in particular, greater relative to the magnitude of the signal noise present. The overall power consumption is reduced as a result of the increased sensing area, since the signal from the woven fabric sensor does not need to be amplified, or not as much. It is also possible to set a first cutoff threshold value so that, for example, a processor is only activated when the signal value generated by the woven fabric sensor is above the first cutoff threshold value.

Overall, the woven fabric sensor according to the invention enables a change in the magnetic field to be detected with high sensitivity. In particular, the proximity of a fingertip can be measured by capacitance as long as there is no contact with the fabric, and the pressure applied can be measured by resistance when there is contact between the fingertip and the fabric sensor.

Exemplary products in which the woven fabric sensor could be integrated are fabrics for clothing, for car seats, for car seat covers, for steering wheels and steering wheel covers, for bedsheets, for carpets, for flooring, for buttons such as turn on/off buttons and/or for switches. The woven fabric sensor could therefore be used in many different products, in particular in the automotive field and/or for healthcare sectors.

Further, woven fabrics comprise less stretch than knitted fabrics. In a woven fabric, the warp runs in a direction of warp and the weft runs along a direction of weft. In general, the warps of a plurality of warps runs parallel to each other and the wefts of a plurality of wefts runs parallel to each other. During manufacture of the woven fabric, the warp is held stationary under tension while the transversing weft is drawn through and inserted over and under the warp. Hereby, the tension of the warp and/ or the weft can be alternated. Different tensions in the warp and weft allows tighter or looser weaving of the conductive yarns which also has the effect of increasing or decreasing the dynamic (non-binary) electrical resistance of the fabric sensor. This allows to customize specifications such as resistance values.

The most common woven fabric type is called plain weave and has the direction of warp arranged perpendicular to the direction of weft. This style of weaving has the weft alternating over and under the warp. The next adjacent weft follows in a mirrored order when passed over and under the warp. Thereby, the warp is fixed by the weft and separated from each other. In the present invention, as described above, the weft passes over or under at least two adjacent warps. Preferably, the at least one adjacent weft passes over or under the warps in a mirrored sequence to the previously described adjacent weft. The warps comprised within a bridge path are thus pressed together. According to a preferred embodiment, the bridge path comprises between three to ten warps, thus, increasing the bridge length. In particular, three or more bridge paths allow to extend with an adjacent bridge path along two ways.

Alternatives plain weave are known as twill weave or sating weave. Plain weave is the preferred embodiment, but it is also possible to implement the woven fabric sensor as described above based on other styles, such as twill weave or sating weave.

Multi-dimensional sensing refers to sensing parameters including, but not limited to, proximity, capacitance, temperature, humidity, vibration, applied force, and pressure differential. In general, multi-dimensional sensing utilizes for example electric field sensing or the Hall Effect for sensing, which are sensitive to a change in the magnetic field.

Electrically conductive materials comprise in particular semiconductors, nickel, copper and/or stainless steel. Stainless steel is a cheap but less conductive mixture comprising nickel and copper achieving optimum signal strength for woven fabric sensors. The non-conductive material could be made of polyamide (PA), in particular polyamide 6.6, also known as Nylon, or recycled polyethylene terephthalate (PET) or a bio-based yarn, such as a yarn made of sugar cane.

Preferably, the woven fabric sensor comprises a first weft having a first bridge section for generating a first bridge path and a second weft having a second bridge section for generating a second bridge path. The first bridge path and the second bridge path are electrically connected to at least one common electrically conductive warp.

Adjacent wefts increase the width of the bridge section along the direction of warp. By increasing the width, the contact surface of two or more adjacent warps is increased. The connection is thereby less susceptible to disconnection by separation of the warp during movement of the fabric, allowing a more reliable sensor. An even further improved sensor can be made by having four, more preferably 6 and most preferably 10 or more adjacent bridge sections comprising the same warps.

Preferably, the second bridge path is electrically connected to at least one electrically conductive warp, which is not electrically connected to the first bridge path. The bridge path extends thereby along the direction of warp and the direction of weft. In particular, a new warp is electrically connected which was not previously connected by the first bridge path. Thereby the sensing area is further increased resulting an even more sensitive sensor.

Preferably, the woven fabric sensor comprises a third weft having a third bridge section for generating a third bridge path. The third weft has at least one weft arranged between the third weft and the first weft. The first bridge path and the third bridge path are electrically connected to at least one common electrically conductive warp.

The third bridge path, which fulfils the above condition, allows to form multiple connection points with the same warps along the direction of warp. Thereby ensuring that a long warp is connected throughout multiple times with another warp. The woven fabric becomes thereby more robust over breakages in single warps within the woven fabric sensor, as the warp is connected to a different warp at a section before and after the breakage, thereby, bypassing the breakage.

Preferably, a plurality of overlapping bridge paths form a continuous path of conductivity. The continuous path of conductivity improves the sensing area by interconnecting multiple electrically conductive warps. In particular, the sensitivity is thereby greatly enhanced because the adjacent bridge paths have an overlapping section. Further, the continuous path of conductivity can be arranged to form different patterns. Exemplarily, this pattern could be circular and/or spiral. In a preferred embodiment, the continuous path of conductivity can split into two or more continuous path of conductivity, thereby forming for example a "Y" pattern.

Preferably, the continuous path of conductivity follows a wave like pattern. The wave like pattern preferably extends along the direction of warp. The wave like pattern ensures a multiple crossing of a single warp making the fabric sensor signal less susceptible to breakages of single warps. Further, the wave like pattern is particularly beneficial for electromagnetic interference. It increases the electromagnetic compatibility. Preferably the wave like shape is of constant nature, in particular comprises a constant frequency and a constant amplitude.

Preferably, the continuous path of conductivity defines a sensor section. The sensor section is preferably bound by a first boundary and a second boundary. The first boundary and the second boundary can be the first warp and the last warp of the plurality of electrically connected warps via one or more continuous paths of conductivity. With other words, the most external warps electrically connected with each other form the boundary. The definition of the sensor section allows the allocation of a signal received by a control unit. This allocation is possible as each section is positioned at a predefined place. As soon as a signal from this section is generated the control unit processing the signal can allocate the signal to the corresponding section, and consequently allocating the sensed signal.

Preferably, a single section comprises at least two paths of conductivity having preferably a wave like pattern. The two paths of conductivity are electrically connecting at least one common electrically conductive warp. This further increases the sensing section, resulting in an even larger sensing area. The increased sensing area provides an even more sensitive sensor.

Preferably, the woven fabric sensor comprises a plurality of sensor sections. The plurality of sensor sections enable a multiple of signals generated by a single woven fabric sensor. This could be utilised, for example, for measuring different interactions by the single woven fabric sensor.

Preferably, the woven fabric sensor comprises a single contact point for transmitting a single output signal. Further preferably, the single contact point is arranged within the sensor section. The single contact point provides a very simple implementation of the invention. As all the warps are connected via the bridge paths, the single contact point can be located anywhere within the defined sensor section. This allows for a wide variety of applications. For example, for a seat cover comprising a woven fabric sensor the single contact point can be allocated at a position that allows enough installation space for the wire to be connected to a control unit or a wireless transmitter. Thereby, failure modes are reduced, the industrial implementation is enhanced and the product can be much easier assembled.

A second aspect of the invention is directed at a multi-dimensional sensing system for measuring sensing parameters comprising: a woven fabric sensor as previously described, and a connector connected to the single contact point for transmitting the single input signal to a control unit. The control unit is configured to process the single input signal. Preferably, the control unit is configured to respond in real time due to the woven sensor signal, in particular including various multi-dimensional sensing parameters, according to the invention. Responding in real time allows post processing to be made redundant and reduces latency limitations caused by post processing filtering. Further, the system can be integrated into an internet of things architecture.

A third aspect of the invention is directed at a seat characterised by a woven fabric sensor as described above. A fourth aspect of the invention is directed at a bed characterized by a woven fabric sensor as described above. The seat, in particular for the automotive industry, or the bed, in particular for healthcare appliances, require an ever increasing amount of sensors for enabling sensing of in particular human physical parameters, human-machine interaction, and human behaviour. A fifth aspect of the invention is directed at an operating method for analysing a single output signal generated by a woven fabric sensor as described above or a multi-dimensional sensing system as described above carried out by a control unit. The method comprising the followings steps: a) Receiving a single input sensor signal having a signal value. b) Analysing the signal value for determining a magnitude of a sensing parameter. c) Generating an output signal indicating the magnitude of the sensing parameter.

Preferably, the step of analysing allows differentiation between a plurality of different information sensed by the woven fabric sensor according to the invention. For example, it is possible to use the woven fabric sensor to detect information about a person's heart rate as well as breathing rate from a single woven fabric sensor having only a single point of contact. The heart rate is for example sensed by use of capacity sensing and the torso expansion, directly correlated to breathing rate, is measured by resistance sensing. Further, the differentiation is made possible by applying a filter to the sensor signal or by performing a Fourier transform to identify different frequencies and amplitudes in the signal relating to heart rate or respiratory rate respectively. Further, the output signal can be used to initiate or trigger a response based on the output signal.

Preferably, a first output signal is generated, when the signal value is greater than a first cutoff threshold value; and/or a second output signal is generated, when the signal value is greater than a second cutoff threshold value; and/or a third output signal is generated when the signal value is greater than a third cutoff threshold value.

The first cutoff threshold reduces the power consumption when the fabric sensor is not in use. In particular, as some magnetic field or electric field is at most times present in the environment. However, when used as a sensor it is not the intention to use it to measure any magnetic field, but, exemplarily only when a finger is approaching the fabric sensor that will change and/or influence and/or disrupt the magnetic or electric field.

The use of a first cutoff threshold can be used as the sensing area of the woven fabric sensor provides a very sensitive signal having a high amplitude. Thereby the first cutoff threshold can be set relatively high, such that unintentional signal outputs are avoided. Further, the cutoff thresholds can be adjusted according to the intended use. For example, a baby in a childseat generates a much lower amplitude signal and requires lower cutoff thresholds, whereas an adult generates a much higher amplitude in the signal resulting in a higher cutoff threshold value.

Preferably, the sensing parameter is any one or multiple of: proximity, capacitance, temperature, humidity, vibration, applied force, or pressure differential.

A sixth aspect of the invention is directed at a computer program product comprising instructions which, when the program is executed by a control unit, cause the control unit to carry out the steps of the operating method described above.

In the following, a multi-dimensional sensing system comprising a woven fabric sensor and a corresponding operating method as well as further features and advantages are explained in more detail with reference to embodiments which are shown schematically in the figures. Herein, show:
- Fig. 1: a woven fabric sensor;
- Fig. 2: an enlarged view of section A of Fig. 1;
- Fig. 3: an enlarged view of the cross-section along line B - B of Fig. 1;
- Fig. 4: an enlarged photograph of a section of a woven fabric sensor;
- Fig. 5: a further enlarged photograph of a section of a woven fabric sensor;
- Fig. 6: a multi-dimensional sensing system including a woven fabric sensor;
- Fig. 7a: an input signal according to the prior art;
- Fig. 7b: an input signal generated by us of a woven fabric sensor according to the invention;
- Fig. 8: a seat including a woven fabric sensor according to the invention;
- Fig. 9: a bed including a woven fabric sensor according to the invention
- Fig. 10: an image of the bed of Fig. 9 generated by the use of the output signal of the woven fabric sensor.

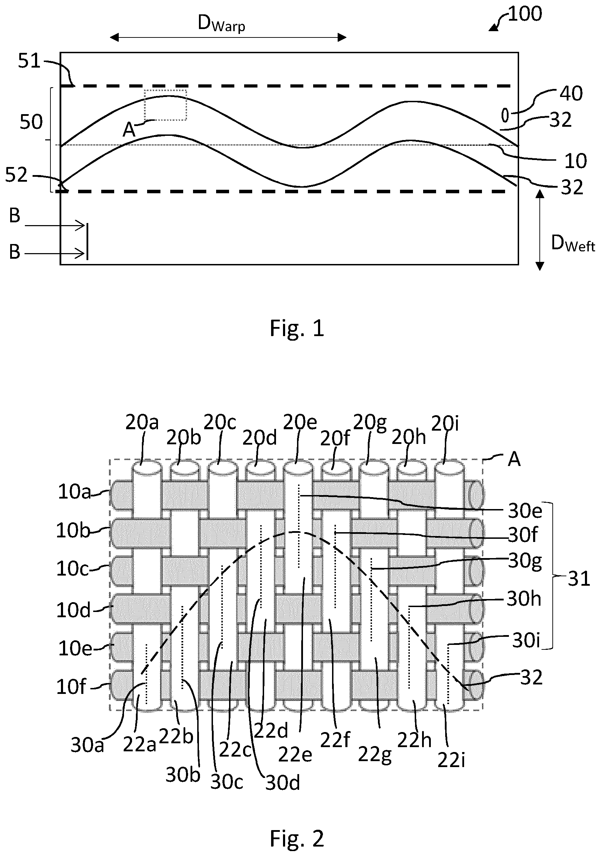

Fig. 1 shows a woven fabric sensor 100 comprising a plurality of electrically conductive warps 10 and electrically non-conductive wefts 20 floating over or under the warp. The woven fabric sensor 100 extends along a direction of warp **D**_{Warp} running parallel to the warp 10 and a direction of weft D_{Weft} running parallel to the weft 20. In the embodiment shown in Fig. 1, the direction of warp **D**_{Warp} is perpendicular to the direction of weft D_{Weft} similar to a plain weave.

The woven fabric sensor 100 has at least one sensor section 50 including at least one continuous path of conductivity 32 electrically connecting the plurality of electrically conductive warps 10 included in the sensor section 50. In Fig. 1, the sensor section 50 exemplarily shows two continuous paths of conductivity 32 within a single sensor section 50. This is enabled because both paths of conductivity 32 have at least one common electrically conductive warp 10, indicated in Fig. 1 by a dotted line in the centre of the sensor section 50.

The sensor section 50 is bound by a first boundary 51 and a second boundary 52. The first and second boundaries 51, 52 are defined by the outermost warp being electrically connected to the continuous path of conductivity 32. The warps 10 arranged within the sensor section 50 are electrically conductive. The warps outside the sensor section 50 can be electrically non-conductive.

The sensor section 50 further comprises a single contact point 40, which is designed to be connected to a connector 210, which is described in more detail below.

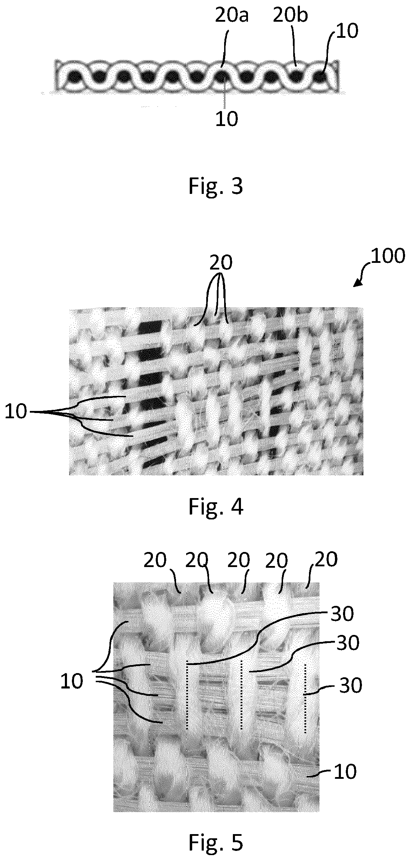

Fig. 2 shows an enlarged view of section A of Fig. 1. In this view, six parallel warps 10a to 10f and nine parallel wefts 20a to 20i are shown. In general, each weft 20a to 20i flows alternately over and under the warp 10a to 10f as shown in Fig. 3, thereby separating the electrically conductive warps 10 from each other. In the section of the weft 20a to 20i defining a bridge path 30a to 30i, the weft 20a to 20i flows over or under at least two adjacent electrically conductive warps 10a to 10f, thereby pressing the comprised warps 10 together to form an electrical connection as best shown in Fig. 4 and Fig. 5.

The consecutive bridge paths 30a, 30b; 30b, 30c have at least one common electrically conductive warp 10e, 10f; 10d, 10e. All bridge paths 30a to 30i are preferably of the same length. Further, the abutting bridge path comprises at least one electrically conductive warp which is not comprised by the adjacent bridge path. The adjacent bridge paths 30 to 30i comprise an overlapping section. The adjacent bridge paths 30 to 30i can be referred to as plurality of overlapping bridge paths 31 forming the continuous path of conductivity 32. As shown in Fig. 1 the continuous path of conductivity 32 is arranged such that it follows a wave like pattern. Adjacent can also be understood as equivalent to abutting.

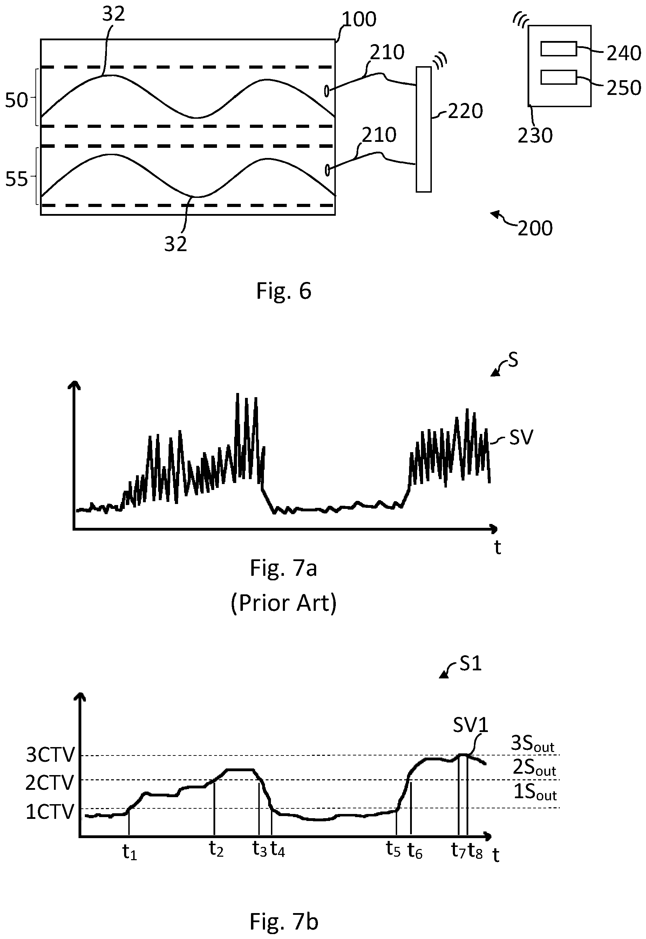

Fig. 6 shows a multi-dimensional sensing system 200. The sensing system 200 comprises a woven fabric sensor 100, as described above, a connector 210 connected to the single contact point 40 and a transmitter 220, which is preferably a wireless transmitter 220, and a control unit 230 comprising a memory 250 and a processor 240 configured to analyse signals sensed by the woven fabric sensor 100.

The woven fabric sensor 100 shown in Fig. 6 exemplarily comprises two sensor sections 50, 55, each connected via a connector 210 and a wireless transmitter 220 to a control unit 230.

The control unit 230 could also be a microcontroller, a computer or a centrally located computer configured to receive from a plurality of woven fabric sensors 100 or sensor sections 50, 55 input signals.

An exemplarily sensor input signal S having a signal value SV according to the prior art, WO 2018 076 106 A1, is shown in Fig. 7a. The sensor signal value SV comprises relatively a large amount of noise in the signal.

The sensor signal of a single input sensor signal Sᵢₙ1, as it is measured with the woven fabric sensor 100, is shown by way of example in Fig. 7b. The signal contains less signal noise.

Further, Fig. 7b shows three cutoff threshold values 1CTV, 2CTV, 3CTV. If the signal value SV1 is below the respective cutoff threshold value, no corresponding output signal 1Sₒᵤₜ, 2Sₒᵤₜ, 3Sₒᵤₜ is generated by the control unit 230.

In Fig. 7b no output signal is generated until t₁. Once the signal value SV1 has risen to a value equal to or greater than the first cutoff threshold value 1CTV, a first output signal 1Sₒᵤₜ is generated until the signal value SV1 has risen to a value greater than the second cutoff threshold value 2CTV at time t₂. This range could be adjusted such that a proximity of a finger is sensed, but, no contact with the woven fabric sensor 100 has occured.

From time t₂ to t₃ the signal value SV1 has risen above the second cutoff threshold value 2CTV so that a second output signal 2Sₒᵤₜ is generated. This range could be adjusted so that a force applied by a finger to the woven fabric sensor 100 is sensed.

Time t₃ to t₄ show a transmission when the finger has been moved away from the woven fabric sensor 100. In this range the first output signal 1Sₒᵤₜ is generated until the signal value SV1 falls below the first cutoff threshold value 1CTV and no output signal is generated.

At time t₅, a force is applied to the woven fabric sensor 100 equivalent to time t₁ to t₂. Between t₆ to t₇, the force applied to the woven fabric sensor 100 increases such that the signal value SV1 increases from t₇ to t₈ to a value above the third cutoff threshold value 3CTV such that a third output signal 3Sₒᵤₜ is generated. The third output signal 3Sₒᵤₜ could be configured to indicate a high force applied to the woven fabric sensor 100.

The three thresholds shown are for demonstration purposes only and could include a number of other thresholds to produce an even more differentiated output signal.

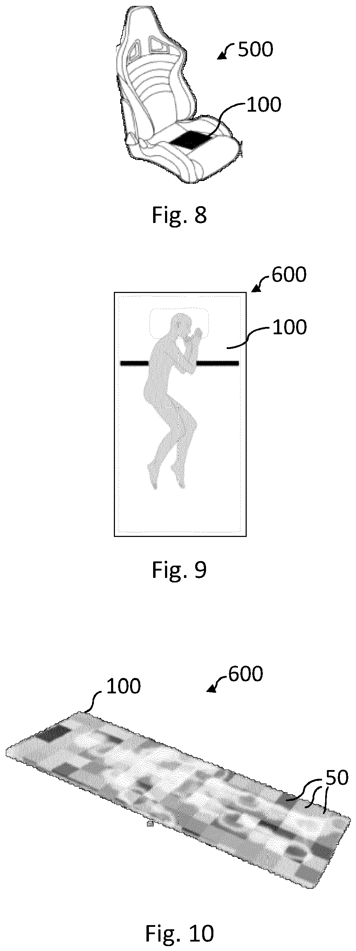

The output signal 1Sₒᵤₜ, 2Sₒᵤₜ, 3Sₒᵤₜ can be used to trigger a predefined action. For example a light bulb of a different colour can be activated for each output signal. It could also be used for a car seat 500 as shown in Fig. 8. The specific location of the sensor section 50, 55 allows, for example, an assessment to be made as to whether the person is sitting out of position (OOP) or is experiencing high levels of stress or other emotional levels which can be correlated with a measurable signal by use of the woven fabric sensor 100.

Another example is a bed 600 as it might be used in the healthcare industry. The sheet consists of a woven fabric sensor 100. The woven fabric sensor 100 comprises a plurality of sensor sections 50. The sensor sections 50 detect in particular whether a person is sleeping stationary or moving on the bed 600.

The woven fabric sensor 100 as described above, as well as the multi-dimensional sensing system 200, can be seamlessly integrated into a woven fabric. The woven fabric sensor 100 is characterised by having an increased sensing area achieved by the use of the described bridge paths 30a to 30e, thereby having an improved sensitivity. The corresponding input signal generated by the woven fabric sensor 100 therefore comprises a higher magnitude in the signal value SV1 compared to standard woven fabric sensors known in the prior art. Also, the relative ratio of signal noise to the absolute signal value SV1 is reduced to a negligible ratio by the woven fabric sensor described above.

### Reference Signs

- 10a to 10f: electrically conductive warps
- 20a to 20i: electrically non-conductive wefts
- 22a to 22i: bridge sections
- 30a to 30e: electrically connecting bridge path
- 31: plurality of overlapping bridge paths
- 32: continuous path of conductivity
- 40: single contact point
- 50, 55: sensor section
- 51: first boundary
- 52: second boundary
- 100: woven fabric sensor
- 200: multi-dimensional sensing system
- 210: connector
- 220: Wireless transmitter
- 230: control unit
- 240: Processor
- 250: Memory
- Sᵢₙ1: single input sensor signal
- SV1: signal value
- Sₒᵤₜ: output signal
- 1Sₒᵤₜ: first output signal
- 2Sₒᵤₜ: second output signal
- 3Sₒᵤₜ: third output signal
- 1CTV: first cutoff threshold value
- 2CTV: second cutoff threshold value
- 3CTV: third cutoff threshold value
- D_{warp}: direction of warp
- D_{weft}: direction of weft

## Claims

1. A woven fabric sensor (100) for multi-dimensional sensing comprising:
a plurality of electrically conductive warps (10a to 10f) arranged parallel to each other, and
at least one electrically non-conductive weft (20a to 20i) floating over or under the warp (10),
wherein the weft (20a to 20i) comprises a bridge section (22a to 22i) floating over or under at least two adjacent electrically conductive warps (10a to 10f) for generating an electrically connected bridge path (30a to 30e).

2. The woven fabric sensor (100) of claim 1, comprising a first weft (20c) having a first bridge section (22c) for generating a first bridge path (30c) and a second weft (20d) having a second bridge section (22d) for generating a second bridge path (30d),
wherein the first bridge path (30c) and the second bridge path (30d) are electrically connected to at least one common electrically conductive warp (10c, 10d).

3. The woven fabric sensor (100) of claim 2, wherein the second bridge path (30d) is electrically connected to at least one electrically conductive warp (10b), which is not electrically connected to the first bridge path (30c).

4. The woven fabric sensor (100) of claim 2 or 3, comprising a third weft (30g) having a third bridge section (22g) for generating a third bridge path (30g),
wherein the third weft (30g) has at least one weft arranged between the third weft (30g) and the first weft (30c),
wherein the first bridge path (30c) and the third bridge path (30g) are electrically connected to at least one common electrically conductive warp (10c, 10d).

5. The woven fabric sensor (100) of any one of claims 1 to 4, wherein a plurality of overlapping bridge paths (31) form a continuous path of conductivity (32).

6. The woven fabric sensor (100) of claim 5, wherein the continuous path of conductivity (32) follows a wave like pattern.

7. The woven fabric sensor (100) of claim 5 or 6, wherein the continuous path of conductivity (32) defines a sensor section (50).

8. The woven fabric sensor (100) of claim 7, comprising a plurality of sensor sections (50, 55).

9. The woven fabric sensor (100) of any one of claims 1 to 8, comprising a single contact point (40) for transmitting a single output signal,
wherein preferably the single contact point (40) is arranged within the sensor section (50, 55).

10. A multi-dimensional sensing system (200) for measuring sensing parameters comprising:
a woven fabric sensor (100) according to claim 9,
a connector (210) connected to the single contact point (40) for transmitting the single input signal to a control unit (230),
wherein the control unit (230) is configured to process the single input signal.

11. A seat (500) **characterized by** a woven fabric sensor (100) according to any one of claims 1 to 9 or a bed (600) **characterized by** a woven fabric sensor (100) according to any one of claims 1 to 9.

12. An operating method for analyzing a single output signal generated by a woven fabric sensor (100) according to any one of claims 1 to 9 or a multi-dimensional sensing system (200) according to claim 10 carried out by a control unit (230), the method comprising the followings steps:
a. receiving a single input sensor signal (Sin1) having a signal value (SV1),
b. analyzing the signal value (SV1) for determining a magnitude of a sensing parameter, and
c. generating an output signal (Sₒᵤₜ) indicating the magnitude of the sensing parameter.

13. The operating method of claim 12, wherein a first output signal (1Sₒᵤₜ) is generated when the signal value (SV1) is equal or greater than a first cutoff threshold value (1CTV), and/or
wherein a second output signal (2Sₒᵤₜ) is generated when the signal value (SV1) is equal or greater than a second cutoff threshold value (2CTV), and/or
wherein a third output signal (2Sₒᵤₜ) is generated when the signal value (SV1) is equal or greater than a third cutoff threshold value (3CTV).

14. The operating method of claim 10 or 11, wherein the sensing parameter is any one of: proximity, capacitance, temperature, humidity, vibration, applied force, or pressure differential.

15. A computer program product comprising instructions which, when the program is executed by a control unit (230), cause the control unit (230) to carry out the steps of the operating method of any one of claims 12 to 14.

## Amended claims

### Amended claims in accordance with Rule 137(2) EPC.

1. A woven fabric sensor (100) for multi-dimensional sensing comprising:
a plurality of electrically conductive warps (10a to 10f) arranged parallel to each other;
at least one electrically non-conductive weft (20a to 20i) floating over or under the warp (10),
wherein the weft (20a to 20i) comprises a bridge section (22a to 22i) floating over or under at least two adjacent electrically conductive warps (10a to 10f) for generating an electrically connected bridge path (30a to 30e); and
a plurality of overlapping bridge paths (31) forming a continuous path of conductivity (32);
**characterized in that** the continuous path of conductivity (32) follows a wave like pattern defining a sensor section (50).

2. The woven fabric sensor (100) of claim 1, comprising a first weft (20c) having a first bridge section (22c) for generating a first bridge path (30c) and a second weft (20d) having a second bridge section (22d) for generating a second bridge path (30d),
wherein the first bridge path (30c) and the second bridge path (30d) are electrically connected to at least one common electrically conductive warp (10c, 10d).

3. The woven fabric sensor (100) of claim 2, wherein the second bridge path (30d) is electrically connected to at least one electrically conductive warp (10b), which is not electrically connected to the first bridge path (30c).

4. The woven fabric sensor (100) of claim 2 or 3, comprising a third weft (30g) having a third bridge section (22g) for generating a third bridge path (30g),
wherein at least one weft is arranged between the third weft (30g) and the first weft (30c),
wherein the first bridge path (30c) and the third bridge path (30g) are electrically connected to at least one common electrically conductive warp (10c, 10d).

5. The woven fabric sensor (100) of any one of claims 1 to 4, comprising a plurality of sensor sections (50, 55).

6. The woven fabric sensor (100) of any one of claims 1 to 5, wherein the wave like pattern comprises a constant frequency and/or amplitude.

7. The woven fabric sensor (100) of any one of claims 1 to 6, wherein the wave like pattern extends along the direction of warp and the continuous path of conductivity (32) crosses at least one electrically conductive warp (10a to 10f) multiple times.

8. The woven fabric sensor (100) of any one of claims 1 to 7, comprising a single contact point (40) for transmitting a single output signal.

9. The woven fabric sensor (100) of claim 8, wherein the single contact point (40) is arranged within the sensor section (50, 55).

10. A multi-dimensional sensing system (200) for measuring sensing parameters comprising:
a woven fabric sensor (100) according to claim 8 or 9,
a connector (210) connected to the single contact point (40) for transmitting the single input signal to a control unit (230),
wherein the control unit (230) is configured to process the single input signal.

11. A seat (500) **characterized by** a woven fabric sensor (100) according to any one of claims 1 to 9 or a bed (600) **characterized by** a woven fabric sensor (100) according to any one of claims 1 to 9.

12. An operating method for analyzing a single output signal generated by a woven fabric sensor (100) according to any one of claims 1 to 9 or a multi-dimensional sensing system (200) according to claim 10 carried out by a control unit (230), the method comprising the followings steps:
a. receiving a single input sensor signal (Sᵢₙ1) having a signal value (SV1),
b. analyzing the signal value (SV1) for determining a magnitude of a sensing parameter, and
c. generating an output signal (Sₒᵤₜ) indicating the magnitude of the sensing parameter.

13. The operating method of claim 12, wherein a first output signal (1Sₒᵤₜ) is generated when the signal value (SV1) is equal or greater than a first cutoff threshold value (1CTV), and/or
wherein a second output signal (2Sₒᵤₜ) is generated when the signal value (SV1) is equal or greater than a second cutoff threshold value (2CTV), and/or
wherein a third output signal (2Sₒᵤₜ) is generated when the signal value (SV1) is equal or greater than a third cutoff threshold value (3CTV).

14. The operating method of claim 12 or 13, wherein the sensing parameter is any one of: proximity, capacitance, temperature, humidity, vibration, applied force, or pressure differential.

15. A computer program product comprising instructions which, when the program is executed by a control unit (230), cause the control unit (230) to carry out the steps of the operating method of any one of claims 12 to 14.
